# EUROPEAN PATENT APPLICATION

(11) **EP 4 659 780 A1**
(43) Date of publication of application: **10.12.2025**
(21) Application number: 24382616.1
(22) Date of filing: 06.06.2024
(51) Int. Cl.: A61M 11/04, A61M 15/06

(54) **ELECTRO-MECHANICAL INHALER DEVICE WITH DOSAGE CONTROL**

(71) Applicant: Nebeln Tech Ltd, 2025 Nicosia Chipre (CY)
(72) Inventor: SAEZ MICO, Carlos, 46530 Puzol (ES); SIMON SOLERA, Javier, 46022 Valencia (ES)
(74) Representative: AAA Law

(57) **Abstract**

The object of the present invention is to provide an electronic vaping device that allows to personalise the composition of the mixture from at least two formulas, being able in turn to monitor the amount inhaled by an adult user or patient, as well as to control its use.

The invention is identified as a device belonging to the sector of electronic cigarettes or vaping devices or inhalers which, as a main differentiating feature with respect to other solutions on the market, allows the customisation of the mixture of various solutions, even with a high viscosity, when exposed to heat emitted by a resistor.

The customisation of this liquid solution is possible thanks to the ability to control the quantity transferred to the atomiser from at least two vial-type cartridges from which the liquid is extracted in a mechanically or electronically controlled manner.

In the case of solutions with a high viscosity, the device with at least one resistor located around the cartridge allows the solution to be exposed to a thermal gradient with the aim of lowering its viscosity to the optimum point to facilitate its passage through the channel for injection into the mixing tank or directly onto the filter or wick.

The filter which will be made of cotton or a highly capillary material such as cellulose, cellulose acetate, porous ceramic materials, zeolites or perforated silicone sponges among others, may be previously impregnated with a flavouring solution, offering a multitude of options, and guaranteeing that the flavour of each puff does not change depending on the dose injected.

The content of these cartridges, as well as the amount provided by each one for mixing, will result in the formula to be vaporised and inhaled by the user. In this way, in case the cartridges contain a THC or CBD-based solution plus an excipient, or any substance or diluted active ingredient or extract, in any proportion, it will be possible to predict the composition of the mixture as well as the amount inhaled.

The device, in addition to the ratio of the mixture, may allow the quantity inhaled to be monitored by establishing a relationship between the amount of air flow and the temperature reached by the resistance to which the formula derived from the mixture is exposed.

This information, together with all the information relating to the patterns of use of the device, may be displayed on the device itself or on other auxiliary mobile devices by means of a wireless connection via wifi, bluetooth or any other device that allows data transfer.

Thus, the present invention details a device considered "intelligent" as it can, among a myriad of possibilities, obtain information on the effects of inhaling a substance, previously identified and with a controlled dose, by obtaining previous information from a smart watch such as heart rate, blood oxygen level, sleep quality, and other aspects relating to the user's health during use and afterwards.

All this information will be processed in the cloud because the device subject of the invention or the linked peripheral devices will be connected to the internet. In this way, the impact of the use of the device on the patient can be monitored, leaving the information available in a unique portal for each user where access codes will be required to enter.

This communication can be bidirectional. That is, from the device to the cloud and from the cloud to the device, which can be established directly between the two or using a peripheral device as a bridge through an app.

It will be in this case, where the device has bidirectional communication, when it can be remotely monitored and controlled by third parties. This use case is mainly conceived for people receiving pharmacological treatments under the supervision of a medical professional. The latter will be able to control the use of the substances consumed, for example cannabinoids such as THC or CBD, as well as the composition of the formula generated from mixing the solution of two or more cartridges.

These cartridges will contain a liquid or pasty solution which, depending on its formulation and active ingredients, will have different levels of viscosity.

The homogeneous mixture of the solutions will give rise to the personalised formula, with the aim of favouring the correct dissolution between both, regardless of the percentage of each of them, it will be more efficient if both are at the same viscosity and density levels. Therefore, the density will be controlled when the solution is being prepared, while the viscosity can be reduced prior to its injection into the mixing tank by generating a thermal gradient from an electronically controlled and controlled power resistor that will achieve the necessary viscosity to flow through the connecting channel between the cartridge and the mixer.

## Description

### FIELD OF THE ART

The present invention falls within the field of inhalers, specifically in the category of electronic cigarettes or vapers. It is characterised by its system for dosing and vaporising a personalised liquid formula by means of a resistance and an atomiser, in order to be inhaled by adults.

### BACKGROUND OF THE INVENTION

In the field of inhalation delivery of active substances, various technologies have been developed to meet the needs of users. Among these technologies are metered dose inhalers (MDIs), dry powder inhalers (DPIs), soft mist inhalers (SMIs) and nebulisers.

Metered dose inhalers (MDIs) deliver a specific amount of medication in aerosol form to the lungs, while dry powder inhalers (DPIs) deliver medication in dry powder form. On the other hand, soft mist inhalers (SMIs) deliver medication in a fine mist, and nebulisers subdivide liquid medications into tiny droplets for delivery.

In addition, innovative devices such as smart inhalers, which automatically record relevant information to improve treatment, have emerged.

Outside the medical field, the use of nebulisers has spread to non-pharmaceutical forms of consumption, such as e-cigarettes/vaporisers, and similar devices have been developed in the recreational cannabis sector.

To date, all devices on the market have specific cartridges or refills, either for single use or refillable directly into the atomiser tank.

Among the devices available on the market, the Mode Device, designed to deliver cannabis accurately, and the Syqe Inhaler, which provides precise doses of inhaled cannabinoids, are notable. However, these devices have limitations in terms of customisation of mixtures, connectivity and data tracking.

In addition, there are relevant patents in the field, such as ES1246010U, which describes a dual tank vape device comprising a tank with two chambers for holding two different types of liquid. The device also includes heating means, such as two heating elements, for heating one or both liquids. These reservoirs within the dual tank are separated so that no mixing of these is performed prior to atomisation.

US20210401061A1, contemplates a complete and secure system for authenticating vape device cartridges and controlling their operation based on verified authenticity and US10842953B2, which describes a cannabinoid delivery system that includes a cartridge for containing a substance containing cannabinoids, a flow regulator for controlling the release of the substance, and a controller for managing the operation of the device. It is intended to provide a controlled and precise way of administering cannabinoids for consumption.

The present invention differs from the aforementioned devices and patents because, firstly, it allows for the customised mixing of substances, even with different viscosities, which provides greater flexibility to the user to adapt the dosage according to his needs. In addition, it uses a disposable filter that can be pre-flavoured, and a system for injecting the formulas for mixing and subsequent vaporisation that ensures precise dosing and an optimal inhalation experience.

On the other hand, a key difference is its ability to collate data with other devices and track results in detail. This allows for a more comprehensive monitoring of the user's health and a better understanding of the effects of long-term substance administration to adjust treatment in a personalised way.

In summary, the invention stands out from all solutions available to date for its ability to custom mix solutions even at high viscosity, with a controlled and monitored dosage with detailed collation and tracking of results by connecting with other devices that measure different indices of user health, positioning it as a versatile and valuable tool for both recreational and therapeutic use by connecting with health professionals offering high potential as a medical research platform, a tool for the wellness market.

### DETAILED DESCRIPTION

The device of the invention (1) is equipped with an atomisation system (5) in its upper part. This system consists of a reservoir or tank (7), which houses a channelling and inlet system for the solution. The solution can come from the cartridges (13) and be introduced into the tank (10.1), or directly into the filter or pad (10.2). The latter is in direct contact with the burner (8), either on the outside, inside or both.

The reservoir (7) is connected to the body (6) of the device, ensuring tightness to prevent leakage of the solution. When the different solutions are administered from the cartridges (13) to the reservoir (7) through the inlet system (10.1), they can be mixed by shaking, by dispersion or by dissolution. This is made possible by the resistance system surrounding the cartridge (13), which is exposed to a thermal gradient to reduce its viscosity prior to injection. In this way, different solutions are injected with the same viscosity, allowing homogeneous mixing. The customised solution resulting from this mixing is transferred to the filter or pad (8) from the outside to the inside, using the principle of capillarity.

In the version where the solution from the cartridges (13) is injected directly onto the filter (8), mixing is not achieved by agitating the tank (7). Instead, a capillary principle is used in combination with simultaneous exposure of the injected solutions to a high temperature generated by the burner (9). This burner, depending on the characteristics of the administered formulas, can reach different temperature ranges, electronically controlled, together with the exposure time and the opening level of the air passage, which is provided with a flow meter (12). Both the burner and the flow meter can be activated either mechanically or electronically. This combination allows control of the amount of customised formula inhaled by the user. Consumption data is collected by the device (1) and can be sent, through various communication options, to the application (2) hosted in the cloud.

The filter (8) can be used in its neutral version or pre-saturated at different levels with additives that provide flavouring or flavouring. This allows a pleasant taste to be enjoyed when nebulising the formula, especially when small doses are involved. In addition, this option is useful when the injected and mixed solutions do not contain any flavour.

At the bottom of the vape device is the body (8). This space houses several essential components, such as the cartridge (13), the heating element (14), the air flow meter (12), the ID reader (16), the cavity (18) and the pressure mechanism (12), among others.

The cartridges are inserted by the user into the device, into the corresponding cavity (17). Each cartridge can contain a different composition and has a unique identifier (16), such as an RFID, which makes it recognisable by the device through a reader (15). This identification allows control and data capture, such as substance limitation, burner temperature (9) and resistance temperature (14), verification of product inhalation or recording of the amount consumed per cartridge.

Both the pressure mechanism (18) and the heating element (14) are essential for dosing substances into the tank (7). Each cartridge contains a heating element (14) that generates a thermal gradient, ensuring a suitable temperature at the top of the cartridge to optimise the vaporisation, mixing and injection of the substance into the atomiser. While at the bottom of the cartridge, a lower temperature is applied to avoid altering the properties of the formula. This is achieved through a mesh-wrapped resistance (14) with a greater number of perforations at the bottom or with a helical resistance with varying spacing between its coils.

After preheating to the appropriate temperature, the substances inside the device are ready to be channelled. Either by means of a physical actuator, a button panel or a touch screen incorporated in the device, the user can activate the pressure mechanism (18). This mechanism exerts controlled pressure on the substance in the cartridge, thereby determining the channelled dose and ensuring a consistent and safe vaping experience. This mechanism, more specifically, can be a plunger with a worm-like piston rod of the insulin pen type, a hydraulic cylinder, or a manual pressure system such as a syringe, among any other possible forms that allow the injection of the solution contained in the cartridges.

### BRIEF DESCRIPTION OF THE FIGURES

In order to aid a better understanding of the features of the invention and to complement this description, the following figures, which are illustrative and nonlimiting in nature, are provided as an integral part of this description:

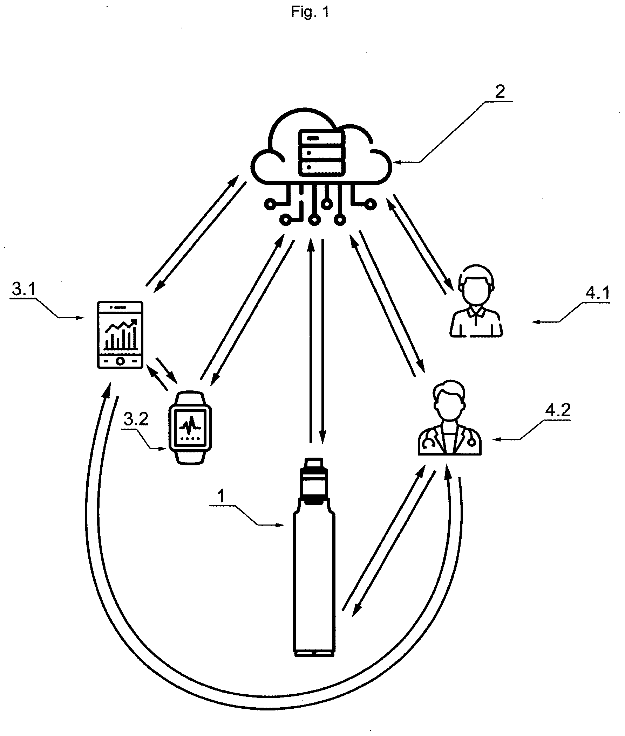

Figure 1 shows the communications logic of the invention.

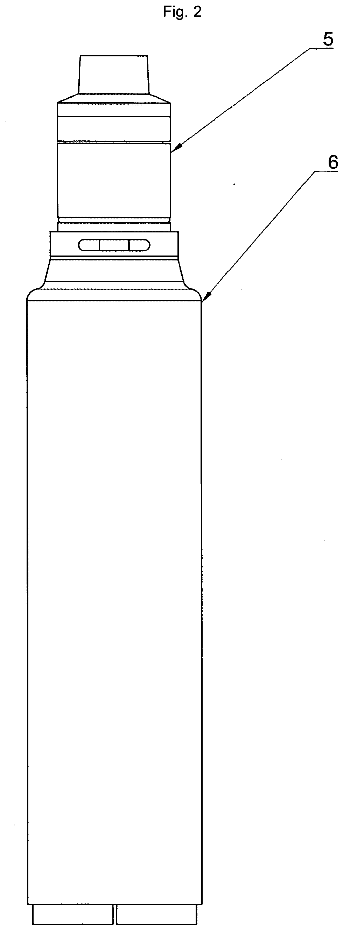

Figure 2 shows a perspective view of the invention where the active parts are highlighted.

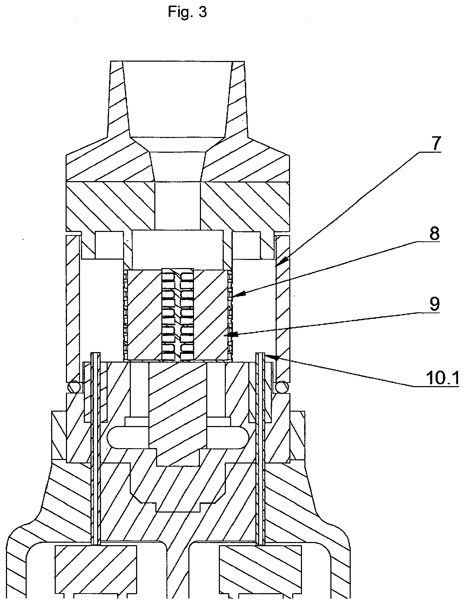

Figure 3 shows a front section view of the elements that make up the atomiser in its version with a mixing tank.

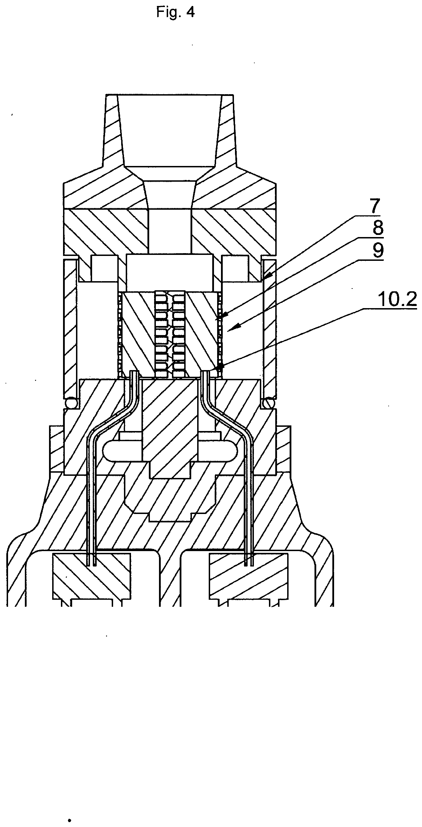

Figure 4 shows a front section view of the elements that make up the atomiser in its version with direct mixing in the filter.

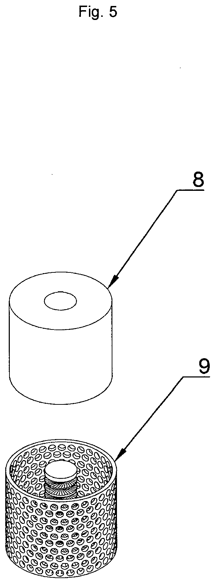

Figure 5 shows a perspective view of the filter and burner.

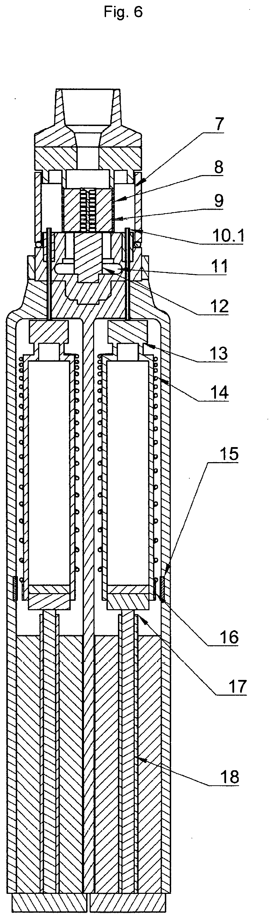

Figure 6 shows a sectional view of the operating system and cartridge housing.

## Claims

1. ELECTRO-MECHANICAL INHALATION DEVICE WITH DOSAGE CONTROL of one, two or more active ingredients, formulations or extracts which may have different viscosities at room temperature, is capable of lowering the viscosity by means of heat transfer through an envelope resistor to the cartridges containing the different formulations to facilitate injection into a reservoir or directly into a filter where the different formulations are mixed and which, depending on the temperature required for atomisation and subsequent inhalation, will be heated by envelope resistors and/or internal to the filter for accurate and personalised delivery. The inhaler consists of two parts: upper part or atomiser and lower part or body. The upper part comprises a reservoir to hold the mixture and containing a filter surrounded by an external and/or internal heater and an air flow regulator by opening control. The lower part or body consists of two or more channels depending on the number of cartridges or vials with a dedicated resistor that together with a reader allows the type of solution to be identified to provide the necessary temperature to reduce its viscosity to be injected by means of a pressure mechanism to the upper part.

2. ELECTRO-MECHANICAL INHALATION DEVICE WITH DOSAGE CONTROL according to claim 1, **characterised by** its capacity to make a mixture of at least two formulations or extracts in any proportion inside a tank or directly on a filter.

3. ELECTRO-MECHANICAL INHALATION DEVICE WITH DOSAGE CONTROL according to claim 2, **characterised by** a sponge-like filter made of cellulose-type material such as cotton, cellulose, cellulose acetate or perforated silicone sponge among other absorbent materials that can be infused with aromas such as terpenes or similar, thus adding a flavouring to the inhalation.

4. ELECTRO-MECHANICAL INHALATION DEVICE WITH DOSAGE CONTROL which according to the previous claim may include the use of filters previously perforated as hollow female cylinders for the insertion of a burner or resistance male threads also in its interior, in addition to the outer resistance, allowing the substance to be heated with greater homogeneity and thus favouring the inhalation of mixtures with a low vaporisation point.

5. ELECTRO-MECHANICAL INHALATION DEVICE WITH DOSAGE CONTROL which, according to claim 3, is **characterised by** allowing the use of ceramic filters or zeolites as a sponge, which allows the inhalation of formulas with a high vaporisation point thanks to their high thermal resistance, which can also be previously infused.

6. ELECTRO-MECHANICAL INHALATION DEVICE WITH DOSAGE CONTROL which, according to the previous claims, allows the control of the temperature exerted on the filter from the resistance, in the version that surrounds it (external) and in the combined version (interior and external resistance), even controlling the temperature of both independently, a feature which, together with the control of the air flow inside the tank at the moment of inhalation, controlled manually or electronically, allows the monitoring of the quantity inhaled by the adult user or patient.

7. ELECTRO-MECHANICAL INHALATION DEVICE WITH DOSAGE CONTROL according to claim 1, **characterised by** a controlled injection system which could be by means of a plunger whose piston rod is a worm screw driven by a thread. Thus, the movement of the thread rotates the plunger in a precise way, drives the cartridge formula allowing a controlled injection.

8. ELECTRO-MECHANICAL INHALER DEVICE WITH DOSAGE CONTROL according to claim 1, **characterised by** a bluetooth, wifi, radiofrequency or any communication system, capable of establishing a bidirectional communication with a mobile device to register, control and measure the use by each user according to the indications of the manufacturer of the formulas, and the doctor's prescription both in terms of dosage and dosage.

9. ELECTRO-MECHANICAL INHALER DEVICE WITH DOSAGE CONTROL according to claim 1, **characterised in that** it can include a labelling system for each cartridge allowing the device to write and/or read and identify each cartridge, in order to obtain a control and monitoring of the consumption.

10. ELECTRO-MECHANICAL INHALATION DEVICE WITH DOSAGE CONTROL according to claim 1, **characterised by** generating a thermal gradient around the cartridge (one, two, or more cartridges), that reduces the viscosity of the solution allowing it to be injected through the channelling system. The thermal gradient is achieved which could be carried out by means of a helical resistance with different spacing between its coils (variable torsion spring type), with a mesh with greater density in its upper part or any other method that allows it, around the cartridge (one, two or more cartridges). The heat at the top, with higher thermal intensity that in the lower part, this will allow greater fluidity, lower viscosity to facilitate injection and passage into the mixing container. Meanwhile, at the upper part, by transmitting less heat through the resistance, the formulation will maintain higher viscosity, yet sufficient to reduce resistance to the push exerted by the pressure mechanism, without affecting the chemical properties of the formulation.

11. ELECTRO-MECHANICAL INHALATION DEVICE WITH DOSAGE CONTROL according to the previous claim, is **characterised by** regulating the optimum requirements for the inhalation of each mixture according to its viscosity, the temperature at which it evaporates and air flow.
